# FASCICULE DE BREVET EUROPEEN

(11) **EP 1 064 102 B1**
(45) Date de publication et mention de la délivrance du brevet: **26.11.2003**
(21) Numéro de dépôt: 99939172.5
(22) Date de dépôt: 09.03.1999
(51) Int. Cl.: B05B 11/06, A61M 15/00

(54) **RESERVOIR, PROCEDE DE REMPLISSAGE DU RESERVOIR ET DISPOSITIF DE DISTRIBUTION DE LA POUDRE CONTENUE DANS LE RESERVOIR**
BEHÄLTER, VERFAHREN ZUR BEFÜLLUNG DIESES BEHÄLTER UND ABGABEVORRICHTUNG FÜR DAS IM BEHÄLTER ENTHALTENE PULVER
RESERVOIR, RESERVOIR FILLING METHOD AND DEVICE FOR DISPENSING POWDER CONTAINED IN THE RESERVOIR

(30) Priorité: 10.03.1998 FR 9802876; 24.06.1998 FR 9808017
(43) Date de publication de la demande: 03.01.2001
(73) Titulaire: VALOIS S.A.S., 27110 Le Neubourg (FR)
(72) Inventeur: BRUNA, Pascal, F-76000 Rouen (FR); GUIFFRAY, Jean-Louis, F-76000 Rouen (FR)
(74) Mandataire: CAPRI
(86) Numéro de dépôt international: FR9900519
(87) Numéro de publication internationale: WO99046055

(56) Documents cités:
- EP-A- 0 407 276
- EP-A- 0 575 239
- EP-A- 0 799 646
- WO-A-91/12895
- WO-A-93/11818
- CH-A- 358 898
- CH-A- 518 744

## Description

La présente invention concerne un réservoir de poudre, un procédé de remplissage de ce réservoir, et un dispositif pour distribuer unitairement une dose de poudre contenu dans ce réservoir à l'aide d'un écoulement d'air sous pression.

En d'autres termes, la présente invention concerne plus spécifiquement un dispositif comportant un ou plusieurs réservoirs distincts prédosés contenant chacun une dose unique de poudre. Lorsqu'il n'y a qu'un seul réservoir, le dispositif est de type monodose. L'invention s'applique toutefois également aux dispositifs de type multidose, par exemple bidose ou quadridose, comportant plusieurs réservoirs prédosés, qui permettent notamment un équilibrage des doses sur les deux narines dans le cas d'une application nasale.

De même, bien que l'invention peut être utilisée avec des produits fluides ou pulvérulents, elle s'applique plus particulièrement à la distribution de doses de poudre, et par la suite il sera fait référence à ce type de produit. En effet, le domaine des produits pulvérulents à distribuer par inhalation, en particulier les médicaments à base de poudre sèche, se développe de plus en plus car les formulations de poudre sèche présentent de nombreux avantages par rapport aux suspensions liquides ou aux solutions liquides. Ainsi, elles sont notamment plus stables et ne nécessitent ni solvant, ni conservateur.

Le document EP-0 407 276 divulgue un distributeur de poudre ou de produit fluide qui comporte un réservoir de produit et une chambre de gaz, en l'occurrence de l'air, ainsi qu'un poussoir déplaçable par rapport au réservoir. Le réservoir comporte un orifice d'entrée et un orifice de sortie, l'orifice d'entrée étant obturé par une membrane qui sépare le réservoir de la chambre de gaz. Ainsi, lorsque le dispositif est actionné, le poussoir est déplacé dans la chambre de gaz, ce qui comprime l'air en aval de la membrane jusqu'à ce qu'un organe de percement solidaire du poussoir vienne percer ladite membrane, ce qui permet à l'écoulement d'air sous pression de traverser la chambre de produit depuis son orifice d'entrée jusqu'à son orifice de sortie, emmenant ainsi la dose de produit avec lui. L'inconvénient principal de ce dispositif est que l'orifice de sortie du réservoir n'est protégé que par le capuchon amovible que l'utilisateur retire avant d'actionner le dispositif. Ainsi, entre l'enlèvement dudit capuchon amovible et l'actionnement du dispositif, il y a un risque de contamination du produit et/ou de perte de ce produit en cas de mauvaise manipulation du dispositif.

Pour résoudre ce problème, il a été proposé des dispositifs de distribution dans lesquels l'orifice de sortie du réservoir est également obturé par une membrane. Ainsi, lorsque le distributeur n'est pas utilisé, l'orifice d'entrée et l'orifice de sortie sont obturés de manière étanche et le produit contenu dans le réservoir ne risque ni de se contaminer, ni de se perdre. Ce n'est qu'au moment de la distribution du produit que les orifices d'entrée et de sortie sont dégagés permettant ainsi le passage de l'écoulement d'air qui chasse la dose de produit vers l'extérieur. Ce genre de distributeur trouve une application privilégiée dans le domaine de la distribution de produits pharmaceutiques sous forme de poudre notamment par administration nasale. Un tel dispositif est notamment divulgué dans le document WO 93/11818.

Un problème qui se pose avec ce type de dispositif concerne le percement de la membrane qui obture l'orifice de sortie du réservoir. En effet, il arrive fréquemment que la membrane ne dégage pas totalement l'orifice de sortie du réservoir, de sorte que du produit peut rester accumulé à l'intérieur du réservoir sur des parties de la membrane qui restent au niveau de l'orifice de sortie. Il s'ensuit que la totalité de la dose de produit n'est pas distribuée ce qui, notamment dans le cas de traitement médical, n'est généralement pas acceptable car une distribution précise et totale de la dose contenue dans le réservoir est souvent une condition pour l'efficacité du traitement.

Un but de la présente invention est de fournir un réservoir de poudre et un dispositif de distribution qui ne présentent pas les inconvénients cités ci-dessus.

La présente invention a notamment pour but de fournir un dispositif de distribution de poudre qui assure une distribution totale et précise de la dose de produit contenue dans le réservoir.

La présente invention a aussi pour but de fournir un dispositif de distribution de poudre qui assure une distribution finement pulvérisée de la dose de produit contenu dans le réservoir.

La présente invention a également pour but de fournir un réservoir de poudre et un dispositif de distribution qui soient simples et peu coûteux à fabriquer et à assembler.

La présente invention a encore pour but de fournir un réservoir de poudre et un dispositif de distribution qui empêchent tout risque de contamination de la dose de produit avant son administration à l'utilisateur du dispositif.

La présente invention a également pour but de fournir un dispositif de distribution de poudre qui peut être utilisé plusieurs fois avec des réservoirs prédosés différents.

La présente invention a aussi pour but de fournir un procédé de remplissage d'un tel réservoir qui soit le plus simple et le plus économique possible.

La présente invention a donc pour objet un réservoir de produit contenant une dose unique de poudre, le réservoir comportant une entrée d'air et une sortie de poudre, ladite entrée d'air comportant un organe de retenue de poudre pour maintenir la poudre dans le réservoir jusqu'à la distribution de la poudre, et ladite sortie de poudre étant obturée, de préférence de manière étanche, par une bille de fermeture qui est expulsé de sa position d'obturation par un écoulement d'air créé lors de la distribution de la poudre.

De préférence, l'organe de retenue de poudre est perméable à l'air.

Avantageusement, l'organe de retenue de poudre est une grille dont les ouvertures sont de dimensions inférieures à la taille de particule de la poudre.

Avantageusement, l'entrée d'air du réservoir est obturée par une membrane de fermeture étanche à la poudre et à l'air.

De préférence, le réservoir comporte en aval de ladite membrane, dans le sens d'écoulement de l'air, ladite grille perméable à l'air et étanche à la poudre, pour retenir la poudre dans le réservoir après ouverture de la membrane de fermeture.

De préférence, ladite bille est expulsée de sa position d'obturation lorsqu'une pression minimale prédéterminée est créée dans le réservoir par ladite chasse d'air.

Selon un mode de réalisation préféré, le réservoir comporte un manchon cylindrique pour recevoir la dose de poudre, ledit manchon incorporant ladite une ouverture d'entrée d'air et ladite ouverture de sortie de poudre, un capuchon cylindrique étant emmanché de manière étanche autour du manchon cylindrique, ledit capuchon étant ouvert du côté de la sortie de poudre et comportant des moyens de fixation de bille pour maintenir fixement ladite bille avant sa mise en place dans sa position d'obturation dans le manchon, et après son expulsion par l'écoulement d'air.

Avantageusement, lesdits moyens de fixation de bille du capuchon comportent des nervures disposées sur la paroi intérieure du capuchon, de sorte qu'après son expulsion de sa position d'obturation, la bille est maintenue fixement dans le capuchon de manière centrée, permettant un écoulement optimal du mélange air/poudre autour d'elle.

La présente invention a aussi pour objet un dispositif de distribution de poudre comportant une chasse d'air pour générer un écoulement d'air lors de l'actionnement du dispositif, et au moins un réservoir.

De préférence, la chasse d'air comporte un piston obturant, de préférence de manière étanche, et coulissant dans une chambre reliée à ladite entrée d'air du réservoir, ledit piston étant relié à un organe d'actionnement actionné par l'utilisateur.

En variante, la chasse d'air comporte un soufflet relié à ladite entrée d'air du réservoir.

De préférence, la sortie de poudre du réservoir est reliée, de préférence de manière étanche, à un canal de sortie dont le diamètre est supérieur au diamètre de la bille, ledit canal comportant des moyens d'arrêt de la bille pour empêcher l'expulsion de celle-ci hors du dispositif lors de l'expulsion de la poudre.

De préférence, l'entrée d'air du réservoir est obturée par une membrane de fermeture étanche à la poudre et à l'air, le dispositif comportant des moyens d'ouverture de l'entrée d'air du réservoir adaptés à ouvrir ladite membrane avant l'expulsion de la dose de poudre.

Avantageusement, lesdits moyens d'ouverture de l'entrée d'air du réservoir comportent un pointeau de percement adapté à percer ladite membrane de fermeture et à relier ledit réservoir à ladite chasse d'air.

Selon un mode de réalisation particulier de l'invention, ladite chasse d'air comporte des moyens de réarmement, de telle sorte que le dispositif est rechargeable et/ou réutilisable.

Avantageusement, les moyens de réarmement comportent un ressort ramenant le piston et l'organe d'actionnement vers leurs positions de repos respectives.

Avantageusement, ledit réservoir est monté de manière amovible dans ledit dispositif, de telle sorte qu'il peut être remplacé après chaque actionnement du dispositif.

Eventuellement, avant l'actionnement, l'ensemble du dispositif est emballé dans un emballage étanche à l'air.

La présente invention a également pour objet un procédé de remplissage d'un tel réservoir de poudre, comportant les étapes de :
- introduire une dose de poudre dans le manchon, à travers l'ouverture de sortie de poudre,
- emmancher le capuchon autour du manchon, ledit capuchon comportant la bille de fermeture maintenue fixement par lesdits moyens de fixation de bille,
- insérer la bille dans l'ouverture de sortie de poudre du manchon.

D'autres caractéristiques et avantages de la présente invention apparaîtront plus clairement au cours de la description détaillée suivante de plusieurs modes de réalisation de la présente invention, donnés à titre d'exemples non limitatifs en regard des dessins joints, sur lesquels :
- la figure 1 est une vue schématique en coupe d'un premier mode de réalisation de la présente invention, avant actionnement,
- la figure 2 est une vue similaire à celle de la figure 1, après actionnement,
- la figure 3 est une vue schématique en coupe d'un second mode de réalisation de la présente invention, avant actionnement,
- la figure 4 est une vue similaire à celle de la figure 3, après ouverture de l'entrée d'air du réservoir et avant expulsion du produit,
- la figure 5 est une vue similaire à celle des figures 3 et 4, après expulsion du produit,
- la figure 6 est une vue schématique en coupe d'un troisième mode de réalisation de la présente invention,
- la figure 7 est une vue schématique en coupe d'un réservoir selon un mode de réalisation avantageux de l'invention,
- les figures 8a à 8d montrent les étapes du procédé de remplissage selon l'invention,
- les figures 9a, 9b et 9c représentent un quatrième mode de réalisation de l'invention, respectivement avant assemblage, avant actionnement et après actionnement, et
- la figure 10 représente un cinquième mode de réalisation de l'invention.

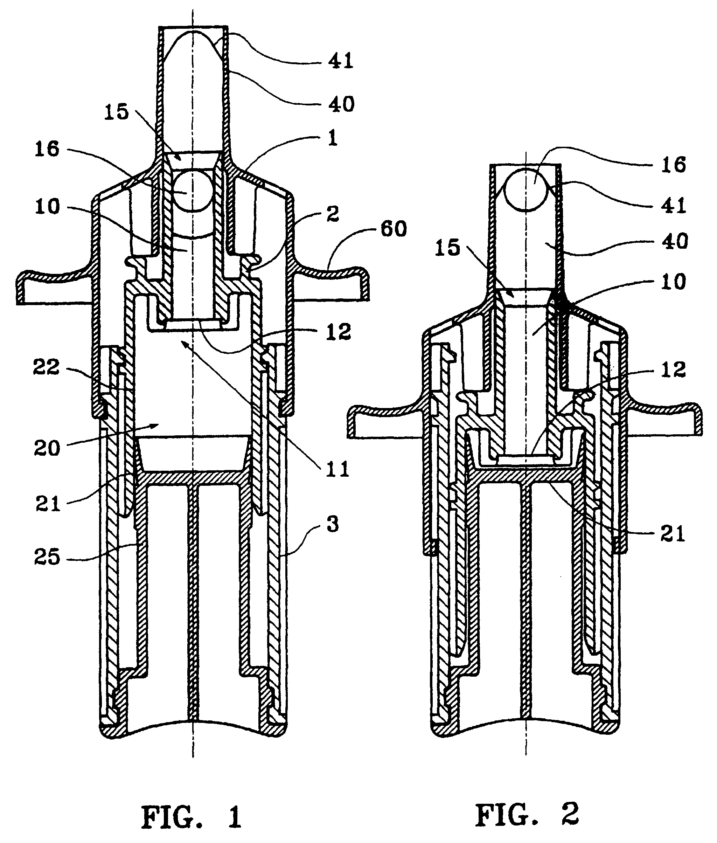

En référence aux figures 1 et 2, il est représenté un premier mode de réalisation de l'invention. Le dispositif comporte un corps supérieur 1 incorporant un canal de sortie 40 et des moyens de prise 60 permettant à l'utilisateur d'actionner le dispositif. A l'intérieur de ce corps supérieur 1 est monté un élément désigné dans son ensemble par la référence numérique 2 qui incorpore le réservoir 10 ainsi que la chambre d'air 22 de la chasse d'air 20. Eventuellement, cet élément 2 pourrait être réalisé d'une pièce avec le corps 1. Un corps inférieur 3 est également prévu pour pouvoir coulisser par rapport au corps supérieur 1 et à l'élément 2, l'utilisateur exerçant une poussée sur le corps inférieur pour actionner le dispositif.

Dans l'exemple représenté sur les figures 1 et 2, le réservoir 10 et la chambre d'air 22 sont donc solidaires dudit élément 2, et un piston 21 solidaire du corps inférieur 3 est disposé de telle manière à obturer et pouvoir coulisser dans ladite chambre d'air 22, de préférence de manière étanche. Le piston 21 peut être solidaire d'un organe d'actionnement 25, ci-après désigné poussoir, fixé audit corps inférieur 3, comme représenté sur les dessins, mais il pourrait aussi être réalisé d'une pièce avec le corps inférieur 3, auquel cas ce dernier agirait en tant qu'organe d'actionnement.

Le réservoir selon l'invention comprend une entrée d'air 11 et une sortie de produit 15. L'entrée d'air 11 comporte un organe de retenue de produit 12 destiné à maintenir le produit dans le réservoir jusqu'à l'actionnement du dispositif. Cet organe de retenue de produit 12 est de préférence réalisé sous la forme d'une grille perméable à l'air, et dont les ouvertures sont de dimensions inférieures à la taille de particules de la poudre. Avantageusement, comme cela est représente sur les dessins, l'entrée d'air 11 du réservoir 10 est directement reliée à la chasse d'air 20, de préférence de manière étanche, de sorte que la totalité de l'écoulement d'air sous pression qui sera créé lors de l'actionnement du dispositif s'écoulera de l'entrée d'air 11 à travers tout le réservoir 10 vers la sortie de produit 15 pour expulser la dose contenue dans le réservoir.

La sortie de produit 15 du réservoir 10 est selon l'invention obturée, de préférence de manière étanche, par une bille de fermeture 16, ou plus généralement par un élément de fermeture 16 de forme sphérique, qui est adapté à être expulsé de sa position d'obturation représentée sur la figure 1 par ledit écoulement d'air qui est créé par l'actionnement du dispositif comme cela sera expliqué ci-dessous.

Ainsi, lorsque l'utilisateur actionne le dispositif, il exerce une pression sur le poussoir 25 de telle sorte que le piston 21 va comprimer l'air contenu dans la chambre 22 de la chasse d'air 20. La grille 12 étant perméable à l'air, la compression de l'air dans la chambre 22 va donc être transmise au réservoir 10 et par conséquent va s'appliquer sur la bille de fermeture 16 qui obture la sortie de produit 15. Les dimensions de la bille de fermeture 16 et sa fixation au niveau de la sortie de produit 15 du réservoir 10 sont réalisés de telle sorte que la bille 16 est expulsée de sa position d'obturation représentée sur la figure 1, lorsqu'une pression minimale prédéterminée est créée par le réservoir 10 dans ladite chasse d'air 20. Ainsi, lorsque cette pression minimale est atteinte, la bille est subitement expulsée vers le canal de sortie 40 du dispositif et l'écoulement d'air créé par la chasse d'air 20 expulse la totalité de la dose contenue dans le réservoir 10. La précompression créée par cette bille de fermeture 16 garantit que lorsque cette dernière est expulsée de sa position d'obturation, l'énergie accumulée dans la main de l'utilisateur est telle que le piston 21 solidaire du poussoir 25 est propulsé dans la chambre 22 créant ainsi un écoulement d'air puissant, c'est-à-dire adapté à finement pulvériser la dose de produit notamment à désagglomérer la poudre. L'écoulement d'air est aussi total, c'est-à-dire que l'ensemble du volume d'air contenu dans la chambre 22 est expulsé sous forme d'écoulement d'air lors de l'actionnement. Toutes les caractéristiques de la distribution de la dose tel que le volume de la dose, le volume et le débit de l'écoulement d'air, sont donc prédéterminées de manière précise et donc très exactement reproductibles. L'efficacité est donc garantie.

De préférence, le canal de sortie 40 a un diamètre supérieur au diamètre de la bille de fermeture 16 pour permettre à la dose de produit d'être expulsé à travers le canal de sortie 40 en s'écoulant autour de la bille 16. De préférence, le canal 40 comporte des moyens d'arrêt ou de fixation 41 de la bille 16 pour empêcher l'expulsion de celle-ci hors du dispositif lors de l'expulsion de la poudre.

L'utilisation d'une bille 16, ou plus généralement d'un élément sphérique, pour fermer la sortie de poudre 15 du réservoir 10 permet de garantir un dégagement complet de cette sortie de produit lors de l'expulsion, de sorte que l'inconvénient existant avec des membranes est évité. De plus, la forme sphérique garantit un bon écoulement de la dose de produit autour de l'élément de fermeture 16 lorsque celui-ci est dans le canal de sortie 40. Ainsi, l'invention permet de garantir une distribution efficace de la totalité de la dose contenue dans le réservoir 10, sans risque de rétention partielle de produit dans le dispositif de distribution.

De préférence, la bille 16 obture de manière étanche l'orifice de sortie 15 du réservoir 10, et cet orifice de sortie est relié de manière étanche au canal de sortie 40. De même, l'entrée d'air du réservoir est de préférence reliée de manière étanche à la chambre d'air 22 de la chasse d'air 20, et le piston 21 obture de préférence de manière étanche ladite chambre d'air 22. De cette manière, avant l'actionnement du dispositif, le produit contenu à l'intérieur du réservoir est complètement séparé de manière étanche de l'atmosphère. Toutefois, il est également envisageable pour renforcer cette étanchéité hermétique du produit contenu dans le réservoir 10 avant l'utilisation du dispositif, d'emballer l'ensemble du dispositif à l'intérieur d'un emballage hermétique (non représenté) qui sera retiré par l'utilisateur juste avant l'utilisation du dispositif.

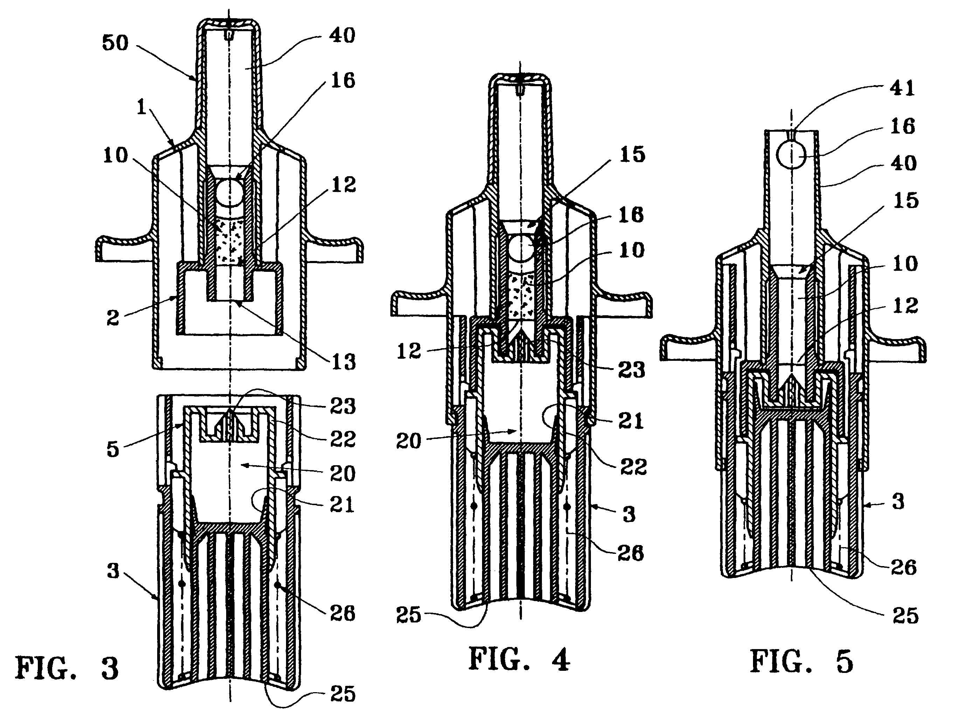

Sur les figures 3 à 5, il est représenté un second mode de réalisation de la présente invention. Les éléments identiques ou similaires au premier mode de réalisation ont été désignés par les mêmes références numériques. La principale différence de ce second mode de réalisation est qu'il prévoit au niveau de l'entrée d'air du réservoir 10, une membrane de fermeture déchirable 13 étanche au produit et à l'air. Pour ouvrir cette membrane de fermeture 13, le dispositif comporte des moyens d'ouverture 23 de l'entrée d'air 10 du réservoir 10 qui sont adaptés à ouvrir ladite membrane 13 avant l'expulsion de la dose de poudre.

Cette membrane de fermeture étanche 13 peut faire office d'organe de retenue 12 du produit. De préférence, comme représenté sue les figures 3 à 5, le réservoir 10 comporte toutefois en plus de la membrane de fermeture 13, un organe de retenue du produit, tel que la grille 12 décrite en référence au premier mode de réalisation. L'avantage dans ce cas est qu'après ouverture de la membrane de fermeture 13 par les moyens de l'ouverture 23, il n'y a aucun risque que du produit s'écoule à l'intérieur de la chambre d'air 22 de la chasse d'air 20. Avantageusement, lesdits moyens d'ouverture de l'entrée d'air comportent un pointeau de percement 23 qui est adapté à percer ladite membrane de fermeture 13 tout en reliant le réservoir 10 à la chasse d'air 20. Dans l'exemple représenté sur les figures 3 à 5, le pointeau de percement 23 comporte des rainures ou alésages permettant de réaliser cette liaison.

Dans le mode de réalisation représenté sur les figures 3 à 5, la chasse d'air, en particulier la chambre d'air 22 n'est plus solidaire du réservoir 10, et l'ensemble du dispositif est réalisé en deux parties, une partie supérieure comportant le corps supérieur 1 et l'élément 2 incorporant le réservoir 10, et une partie inférieure comportant le corps inférieur 3 et un second élément 5 réalisant la chambre d'air 22 et incorporant à son extrémité ledit pointeau de percement 23. La première étape lorsque l'utilisateur veut actionner le dispositif est d'assembler les deux parties du dispositif, comme représenté sur la figure 4. Lors de cet assemblage, le pointeau de percement 23 vient avantageusement percer la membrane 13 qui ferme l'entrée d'air du réservoir 10. La liaison entre les deux parties peut être réalisée de manière quelconque, notamment par encliquetage. De préférence, la liaison entre le second élément 5, qui incorpore la chambre d'air 22, et le premier élément 2, qui incorpore le réservoir 10, se fait de manière étanche lors de l'assemblage des deux parties du dispositif. Lorsque le dispositif est dans sa configuration représentée sur la figure 4, l'expulsion de la dose de poudre est réalisée de la même manière que dans le cas du premier mode de réalisation, c'est-à-dire que l'utilisateur appuie sur le poussoir 25 comprimant ainsi l'air contenu dans la chambre d'air 22 au moyen du piston 21, ladite pression, lorsqu'elle atteint le seuil minimal requis, expulsant la bille de fermeture 16 de sa position d'obturation permettant une expulsion de la dose par l'écoulement d'air.

Comme représenté sur les dessins, le poussoir 25 et le piston 21 peuvent être réalisés d'une pièce, tel qu'un cylindre d'actionnement 25 comportant à son extrémité le piston 21. De même, l'orifice de sortie du canal de sortie 40 peut être obturé par un capuchon amovible 50, comme représenté sur les figures 3 et 4. Bien entendu, le capuchon de fermeture 50 peut s'adapter sur le dispositif des figures 1 et 2, et il pourrait être omis dans le second mode de réalisation si celui-ci était également emballé de manière hermétique dans un emballage (non représenté).

Selon une variante de l'invention, le dispositif peut être réalisé de manière réutilisable, c'est-à-dire qu'il peut être réutilisé plusieurs fois avec des réservoirs prédosés 10 différents. Dans ce cas, le dispositif peut comporter dès l'origine plusieurs réservoirs qui sont successivement vidés lors d'actionnements successifs du dispositif. Il s'agit dans ce cas d'un dispositif multidoses. Concernant les dispositifs monodoses, ils peuvent être conçus de manière rechargeable. Pour ce faire, le réservoir 10 est monté de manière amovible dans le corps 1, de telle sorte qu'il peut être remplacé facilement après chaque actionnement du dispositif. D'autre part, la chasse d'air comporte des moyens de réarmement 26, en particulier un ressort, qui est adapté à ramener le piston 21 et le poussoir 25, et donc également le corps inférieur 3 vers leurs positions de repos respectives. En référence aux figures 3 à 5, l'élément 2 qui incorpore le réservoir 10 est emmanché à force dans le corps supérieur 1 et, après utilisation du dispositif, il peut être facilement remplacé par un nouvel élément 2 qui comporte un réservoir plein par simple séparation des deux parties du dispositif et remplacement de l'élément 2. Il serait également envisageable de réaliser le premier mode de réalisation décrit en référence aux figures 1 et 2 de manière rechargeable.

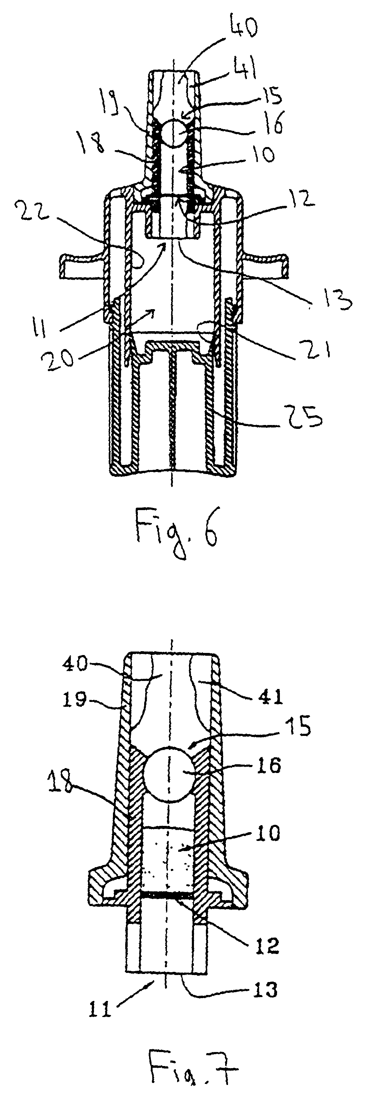

En référence aux figures 6 et 7, il est représenté un autre mode de réalisation avantageux de l'invention, dans lequel le réservoir 10 selon l'invention comprend un manchon tubulaire 18 recevant la poudre, ledit manchon 18 incorporant ladite ouverture d'entrée d'air 11 et ladite ouverture de sortie de produit 15. Comme visible sur les figures, le réservoir 10 de l'invention comporte en outre un capuchon 19 sensiblement cylindrique emmanché ou emboîté autour du manchon 18, ledit capuchon 19 étant ouvert à ses deux extrémités. La partie du capuchon 19 qui dépasse axialement l'ouverture de sortie de produit 15 forme donc une partie du canal d'expulsion 40 du produit. Ce capuchon 19 peut être monté de manière étanche sur le manchon 18 de manière fixe ou amovible d'une quelconque manière appropriée, l'assemblage de ces deux pièces du réservoir devant être suffisamment solide pour résister à l'actionnement du dispositif de distribution.

L'entrée d'air 11 du réservoir 10 comporte de préférence une grille 12 et une membrane 13, comme décrit précédemment. Cette membrane de fermeture étanche 13 peut faire office d'organe de retenue 12 du produit puisqu'elle retient la poudre dans le réservoir. De préférence, le réservoir 10 comporte toutefois et la membrane de fermeture 13, et la grille 12 décrite ci-dessus. L'avantage dans ce cas est qu'après ouverture de la membrane de fermeture 13 par les moyens d'ouverture, il n'y a aucun risque que de la poudre s'écoule à l'intérieur de la chasse d'air.

La partie du capuchon 19 formant canal de sortie 40 a un diamètre supérieur au diamètre de la bille de fermeture 16 pour permettre à la dose de produit d'être expulsé en s'écoulant autour de la bille 16. Selon l'invention, le capuchon 19 comporte des moyens de fixation 41 de la bille 16 pour empêcher l'expulsion de celle-ci hors du dispositif lors de l'expulsion du produit, et pour la maintenir fixement avant et après sa mise en place dans sa position d'obturation. Ces moyens de fixation de bille sont avantageusement réalisés sous la forme de plusieurs nervures 41 réparties sur la périphérie interne dudit capuchon 19, et adaptées à coincer la bille 16 de manière centrée dans le canal d'expulsion 40.

Ainsi, lors de l'expulsion du mélange poudre/air, celui-ci peut s'écouler tout autour de la bille et ainsi former un jet propre, droit et régulier.

Dans le cas d'un emmanchement amovible du capuchon 19 sur le manchon 18, la fixation de bille 16 dans le capuchon 19 après l'expulsion du produit permet d'éliminer le risque de perte de la bille lors d'une éventuelle séparation du capuchon 19 et du manchon 18.

Ces moyens de fixation de bille 41 procurent également un autre avantage qui sera explicité ci-après en référence au procédé de remplissage du réservoir.

En effet, la conception du réservoir de l'invention selon le mode de réalisation des figures 6 et 7 permet de simplifier son remplissage et son assemblage, d'où il en résulte une diminution de coût.

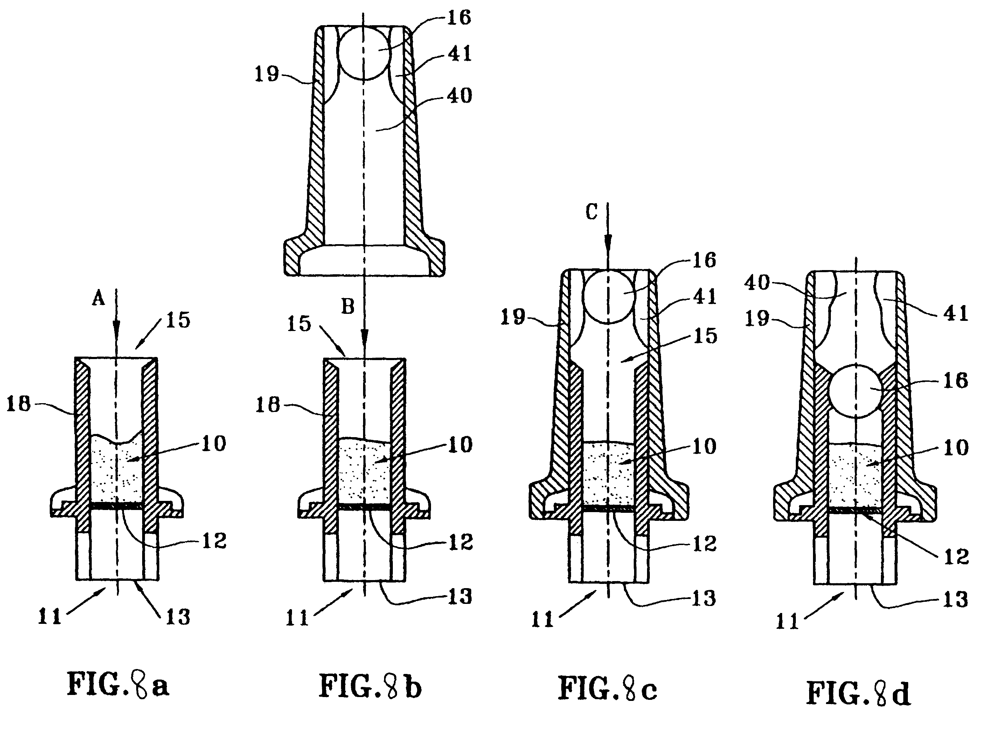

Les étapes successives du procédé de remplissage du réservoir 10 sont représentées sur les figures 8a à 8d.

D'abord, la dose précise de produit est introduite dans le manchon 18, comme représenté par la flèche A sur la figure 1a. La poudre est retenue dans le manchon 18 par la grille 12.

Ensuite, l'unité formée du capuchon 19 et de la bille 16 coincée dans les moyens de fixation de bille 41 du capuchon, est emmanchée autour du manchon 18. Cette étape est représentée par la flèche B sur la figure 1b. Avantageusement, le capuchon 19 et le manchon 18 comportent des moyens de butée respectifs pour définir la position assemblée du réservoir.

Ensuite, la bille 16 est insérée dans l'ouverture de sortie de produit 15 du manchon 18, selon la flèche C sur la figure 1c, pour obturer le réservoir.

Avantageusement, l'entrée d'air 11 est obturée de manière étanche par la membrane 13, et la bille 16 obture la sortie du réservoir également de manière étanche, de sorte qu'après l'étape C ci-dessus, le contenu du réservoir est totalement isolé de l'extérieur.

Les avantages principaux du procédé selon l'invention sont :
- l'assemblage du réservoir se limite au montage de deux pièces, à savoir l'emboîtement de l'unité capuchon/bille sur le manchon. La machine d'assemblage du réservoir est donc simplifiée ; cette simplification est rendue possible par la présence des moyens de fixation de bille 41 dans le capuchon 19 ;
- l'insertion de la bille 16 dans le manchon 18 est facilitée du fait qu'elle est prépositionnée à proximité de l'ouverture de sortie 15, et de manière centrée, lors de l'emmanchement du capuchon 19.

La mise en oeuvre du réservoir selon l'invention, avec le capuchon 19 emmanché autour du manchon 18 obturé par la bille 16, procure donc le double avantage de simplifier le procédé de remplissage et d'assemblage du réservoir, et de garantir une distribution totale et parfaite de la dose de poudre contenue dans le réservoir.

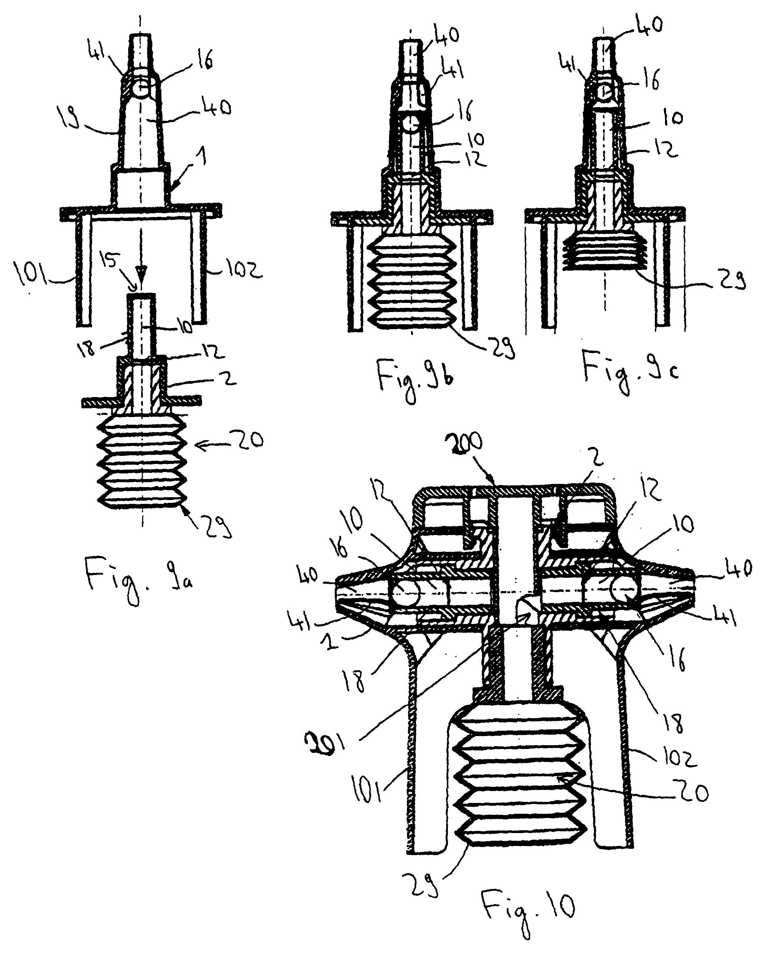

Sur les figures 9a, 9b et 9c, il est représenté encore un autre mode de réalisation de l'invention dans lequel la chasse d'air 20 du dispositif de distribution est réalisée à l'aide d'un soufflet 29 actionné par l'utilisateur. Le fonctionnement d'un tel soufflet 29 pour créer un écoulement d'air est bien connu et ne sera donc pas décrit plus amplement ici. Avantageusement, le corps supérieur 1 comporte deux montants latéraux 101, 102 qui, à l'état monté du dispositif, entourent le soufflet 29 pour empêcher un actionnement accidentel de celui-ci, tout en permettant à l'utilisateur d'appuyer sur le fond du soufflet 29 pour actionner le dispositif et expulser la dose. Le procédé de remplissage décrit en relation aux figures 8a à 8d est applicable ici, comme visible sur les figures 9a et 9B.

En référence à la figure 10, il est représenté encore un autre mode de réalisation de l'invention, dans lequel' le dispositif de distribution est un multidose, en particulier un bidose. Le dispositif comporte ainsi deux réservoirs 10 réalisés selon l'invention, qui dans dans l'exemple représenté sont disposés horizontalement et sont chacun reliés à un canal de sortie 40 respectif. Le dispositif comporte un organe rotatif 200 qui commande un sélecteur d'écoulement 201 adapté à diriger l'écoulement d'air créé par la chasse d'air 20 (ici, le soufflet 29) vers le réservoir 10 choisi par l'utilisateur selon la position dudit organe rotatif 200. Ce type de dispositif peut bien entendu comporter plus de deux réservoirs, et le positionnement de ceux-ci peut aussi être réalisé de manière différente à celle représentée sur la figure 10.

D'autres variantes et modifications sont possibles sans sortir du cadre de la présente invention, et l'invention n'est pas limitée aux exemples de réalisation décrits sur les dessins. En particulier, les modes de réalisation décrits ci-dessus peuvent être combinés entre eux, et les diverses caractéristiques ne sont pas limitées aux exemples de réalisation particuliers pour lesquels elles ont été décrites. D'autre part, il est envisageable d'utiliser un gaz différent de l'air pour créer l'écoulement de gaz sous pression.

## Revendications

1. Réservoir (10) de produit contenant une dose unique de poudre, le réservoir (10) comportant une entrée d'air (11) et une sortie de poudre (15), ladite entrée d'air (11) comportant un organe de retenue de poudre (12 et/ou 13) pour maintenir la poudre dans le réservoir (10) jusqu'à la distribution de la poudre, et ladite sortie de poudre (15) étant obturée, de préférence de manière étanche, par une bille de fermeture (16) qui est expulsé de sa position d'obturation par un écoulement d'air créé lors de la distribution de la poudre.

2. Réservoir selon la revendication 1, dans lequel l'organe de retenue de poudre (12) est perméable à l'air.

3. Réservoir selon la revendication 2, dans lequel l'organe de retenue de poudre est une grille (12) dont les ouvertures sont de dimensions inférieures à la taille de particule de la poudre.

4. Réservoir selon la revendication 1, dans lequel l'entrée d'air (11) du réservoir (10) est obturée par une membrane de fermeture (13) étanche à la poudre et à l'air.

5. Réservoir selon les revendications 3 et 4, dans lequel le réservoir (10) comporte en aval de ladite membrane (13), dans le sens d'écoulement de l'air, ladite grille (12) perméable à l'air et étanche à la poudre, pour retenir la poudre dans le réservoir (10) après ouverture de la membrane de fermeture (13).

6. Réservoir selon l'une quelconque des revendications précédentes, dans lequel ladite bille (16) est expulsée de sa position d'obturation lorsqu'une pression minimale prédéterminée est créée dans le réservoir (10) par ledit écoulement d'air.

7. Réservoir selon l'une quelconque des revendications précédentes, dans lequel le réservoir (10) comporte un manchon cylindrique (18) pour recevoir la dose de poudre, ledit manchon (18) incorporant ladite une ouverture d'entrée d'air (11) et ladite ouverture de sortie de poudre (15), un capuchon cylindrique (19) étant emmanché de manière étanche autour du manchon cylindrique (18), ledit capuchon (19) étant ouvert du côté de la sortie de poudre (15) et comportant des moyens de fixation de bille (41) pour maintenir fixement ladite bille (16) avant sa mise en place dans sa position d'obturation dans le manchon (18), et après son expulsion par l'écoulement d'air.

8. Réservoir selon la revendication 7, dans lequel lesdits moyens de fixation de bille du capuchon (19) comportent des nervures (41) disposées sur la paroi intérieure du capuchon, de sorte qu'après son expulsion de sa position d'obturation, la bille (16) est maintenue fixement dans le capuchon de manière centrée, permettant un écoulement optimal du mélange air/poudre autour d'elle.

9. Dispositif de distribution de poudre, **caractérisé en ce qu'**il comporte une chasse d'air (20) pour générer un écoulement d'air lors de l'actionnement du dispositif, et au moins un réservoir (10) selon l'une quelconque des revendications 1 à 8.

10. Dispositif selon la revendication 9, dans lequel la chasse d'air (20) comporte un piston (21) obturant, de préférence de manière étanche, et coulissant dans une chambre (22) reliée à ladite entrée d'air (11) du réservoir (10), ledit piston (21) étant relié à un organe d'actionnement (25) actionné par l'utilisateur.

11. Dispositif selon la revendication 9, dans lequel la chasse d'air (20) comporte un soufflet (29) relié à ladite entrée d'air (11) du réservoir (10).

12. Dispositif selon l'une quelconque des revendications 9 à 11, dans lequel la sortie de poudre (15) du réservoir (10) est reliée, de préférence de manière étanche, à un canal de sortie (40) dont le diamètre est supérieur au diamètre de la bille (16), ledit canal (40) comportant des moyens d'arrêt (41) de la bille (16) pour empêcher l'expulsion de celle-ci hors du dispositif lors de l'expulsion de la poudre.

13. Dispositif selon l'une quelconque des revendications 9 à 12, dans lequel l'entrée d'air (11) du réservoir (10) est obturée par une membrane de fermeture (13) étanche à la poudre et à l'air, le dispositif comportant des moyens d'ouverture (23) de l'entrée d'air (11) du réservoir (10) adaptés à ouvrir ladite membrane (13) avant l'expulsion de la dose de poudre.

14. Dispositif selon la revendication 13, dans lequel lesdits moyens d'ouverture (23) de l'entrée d'air (11) du réservoir (10) comportent un pointeau de percement (23) adapté à percer ladite membrane de fermeture (13) et à relier ledit réservoir (10) à ladite chasse d'air (20).

15. Dispositif selon l'une quelconque des revendications 9 à 14, dans lequel ladite chasse d'air (20) comporte des moyens de réarmement (26), de telle sorte que le dispositif est rechargeable et/ou réutilisable.

16. Dispositif selon les revendications 10 et 15, dans lequel les moyens de réarmement comportent un ressort (26) ramenant le piston (21) et l'organe d'actionnement (25) vers leurs positions de repos respectives.

17. Dispositif selon l'une quelconque des revendications 9 à 16, dans lequel ledit réservoir (10) est monté de manière amovible dans ledit dispositif, de telle sorte qu'il peut être remplacé après chaque actionnement du dispositif.

18. Dispositif selon l'une quelconque des revendications 9 à 17, dans lequel, avant l'actionnement, l'ensemble du dispositif est emballé dans un emballage étanche à l'air.

19. Procédé de remplissage d'un réservoir de poudre selon la revendication 7 ou 8, **caractérisé en ce qu'**il comporte les étapes de :
- introduire (A) une dose de poudre dans le manchon (18), à travers l'ouverture de sortie de poudre (15),
- emmancher (B) le capuchon (19) autour du manchon (18), ledit capuchon (19) comportant la bille de fermeture (16) maintenue fixement par lesdits moyens de fixation de bille (41 ),
- insérer (C) la bille (16) dans l'ouverture de sortie de poudre (15) du manchon (18).

## Claims

1. A reservoir (10) for a product containing a single dose of powder, the reservoir (10) comprising an air inlet (11) and a powder outlet (15), said air inlet (11) comprising a powder retention device (12 and/or 13) to keep the powder in the reservoir (10) until dispensation of the powder and said powder outlet (15) being closed, preferably in a sealed manner, by a closing ball (16) which is removed from its closing position by a flow of air created at the time the powder is being dispensed.

2. A reservoir according to claim 1, in which the powder retention device (12) is permeable to air.

3. A reservoir according to claim 2, in which the powder retention device is a grid (12) the openings of which have dimensions smaller than the particle size of the powder.

4. A reservoir according to claim 1, in which the air inlet (11) of the reservoir (10) is closed by a closing membrane (13) that forms a seal to both powder and air.

5. A reservoir according to claims 3 and 4, in which the reservoir (10) comprises downstream from said membrane (13), in the direction of the flow of the air, said grid (12) which is permeable to air and which forms a seal to the powder, in order to retain the powder in the reservoir (10) after opening the closing membrane (13).

6. A reservoir according to any one of the preceding claims, in which said ball (16) is removed from its closing position when a minimum predetermined pressure is created in the reservoir (10) by said flow of air.

7. A reservoir according to any one of the preceding claims, in which the reservoir (10) comprises a cylindrical sleeve (18) to receive the dose of powder, said sleeve (18) incorporating said air inlet opening (11) and said powder outlet opening (15), a cylindrical cowl (19) being fitted in a sealed fashion around the cylindrical sleeve (18), said cowl (19) being open on the powder outlet side (15) and comprising means for fixing the ball (41) in order to keep said ball fixed (16) before its placement in its closing position in the sleeve (18) and after its removal by the flow of air.

8. A reservoir according to claim 7, in which said ball fixing means of the cowl (19) comprise fins (41) arranged on the internal wall of the cowl, in such a way that after its removal from its closing position, the ball (16) is held fixed in the cowl in a centered fashion, allowing optimum flow of the air/powder mixture around it.

9. A device for dispensing a powder, **characterized in that** it comprises an air blast (20) to generate an air flow at the time the device is actuated and at least one reservoir (10) according to any one of Claims 1 to 8.

10. A device according to claim 9, in which the air blast (20) comprises a piston (21) blocking, preferably in a sealed fashion, and sliding within a chamber (22) connected to said air inlet (11) of the reservoir (10), said piston (21) being connected to an actuating member (25) actuated by the user.

11. A device according to claim 9, in which the air blast (20) comprises a bellows (29) connected to said air inlet (11) of the reservoir (10).

12. A device according to any one of claims 9 to 11, in which the powder outlet (15) from the reservoir (10) is connected, preferably in a sealed fashion, to an outlet channel (40) the diameter of which is greater than the diameter of the ball (16), said channel (40) comprising stop means (41) for the ball (16) to prevent its expulsion out of the device when the powder is being expelled.

13. A device according to any one of claims 9 to 12, in which the air inlet (11) of the reservoir (10) is closed by a closing membrane (13) that forms a seal to both the powder and the air, the device comprising means (23) of opening the reservoir air inlet (11) suitable for opening said membrane (13) before expulsion of the dose of powder.

14. A device according to claim 13, in which said means (23) of opening the reservoir air inlet (11) comprise a piercing needle (23) suitable for piercing said closing membrane (13) and for connecting said reservoir (10) to said air blast (20).

15. A device according to any one of claims 9 to 14, in which said air blast (20) comprises resetting means (26), such that the device can be recharged and/or reused.

16. A device according to claims 10 and 15, in which the resetting means comprise a spring (26) that returns the piston (21) and the actuating member (25) to their respective stand-by positions.

17. A device according to any one of claims 9 to 16, in which said reservoir (10) is mounted in a detachable manner in said device in such a way that it can be replaced after each actuation of the device.

18. A device according to any one of claims 9 to 17, in which before actuation, the whole of the device is packaged in an air-tight package.

19. A method of filling a powder reservoir according to claim 7 or 8, **characterized in that** it comprises the steps of :
- introducing (A) a dose of powder into the sleeve (18), through the powder outlet opening (15),
- fitting (B) the cowl (19) around the sleeve (18), said cowl (19) comprising the closing ball (16) kept fixed by said ball fixing means (41),
- inserting (C) the ball (16) in the powder outlet opening (15) of the sleeve (18).

## Patentansprüche

1. Behälter (10) für ein Produkt, das eine einzige Pulver-Dosis enthält, wobei der Behälter (10) einen Lufteinlaß (11) und einen Pulver-Auslaß (15) umfasst, wobei der Lufteinlaß (11) ein Rückhalteorgan (12) und/oder (13) für das Pulver aufweist, um das Pulver in den Behälter (10) bis zur Abgabe des Pulvers zu halten, wobei der Pulver-Auslaß (15) vorzugsweise in dichter Weise durch eine Verschlußkugel (16) verschlossen ist, die durch einen Luftstrom, der bei der Abgabe des Pulvers erzeugt wird, aus ihrer Verschlußstellung ausgestoßen wird.

2. Behälter nach Anspruch 1, bei dem das Rückhalteorgan (12) für das Pulver luftdurchlässig ist.

3. Behälter nach Anspruch 2, bei dem das Rückhalteorgan (12) für das Pulver ein Gitter (12) ist, dessen Öffnungen kleinere Abmessungen aufweisen, als die Größe der Pulverteilchen.

4. Behälter nach Anspruch 1, bei dem der Lufteinlaß (11) des Behälters (10) durch eine für das Pulver und Luft dichte Verschlußmembran (13) verschlossen ist.

5. Behälter nach den Ansprüchen 3 und 4, bei dem der Behälter (10) in Richtung der Luftströmung hinter der Membran (13) das für Luft durchlässige und für das Pulver dichte Gitter (12) umfasst, um das Pulver im Behälter (10) nach dem Öffnen der Verschlußmembran (13) zurückzuhalten.

6. Behälter nach einem der vorhergehenden Ansprüche, bei dem die Kugel (16) aus ihrer Verschlußstellung ausgestoßen wird, wenn ein vorbestimmter Minimaldruck im Behälter (10) durch die Luftströmung erzeugt wird.

7. Behälter nach einem der vorhergehenden Ansprüche, bei dem der Behälter (10) eine zylindrische Buchse (18) zum Aufnehmen der Pulver-Dosis umfasst, wobei die Buchse (18) die Lufteintrittsöffnung (11) und die Pulveraustrittsöffnung (15) aufweist und eine zylindrische Kappe (19) in dichter Weise um die zylindrische Buchse (18) herum aufgepreßt ist, wobei die Kappe (19) auf der Seite des Pulver-Auslasses (15) offen ist und Kugel-Befestigungsmittel (41 ) für die Kugel aufweist, um besagte Kugel (16) festzuhalten, bevor sie in ihre Verschlußstellung in der Buchse (18) gebracht wird und nachdem sie durch den Luftstrom ausgestoßen worden ist.

8. Behälter nach Anspruch 7, bei dem die Kugel-Befestigungsmittel der Kappe (19) Rippen (41 ) umfassen, die auf der Innenwand der Kappe derart angeordnet sind, daß die Kugel (16) nachdem sie aus ihrer Verschlußstellung ausgestoßen worden ist, in der Kappe in zentrierter Weise festgehalten wird, wodurch ein optimales Strömen der Luft/Pulver-Mischung um sie herum ermöglicht wird.

9. Pulver-Abgabevorrichtung, **dadurch gekennzeichnet, daß** sie eine Luftausstoßeinrichtung (20) umfasst, um bei der Betätigung der Vorrichtung einen Luftstrom zu erzeugen, sowie wenigstens einen Behälter (10) nach einem der Ansprüche 1 bis 8.

10. Vorrichtung nach Anspruch 9, bei der die Luftausstoßeinrichtung (20) einen Kolben (21) aufweist, der vorzugsweise in dichter Weise abschließt und in einer Kammer (22) gleitet, die mit dem Lufteintritt (11) des Behälters (10) verbunden ist, wobei der Kolben (21) mit einem Betätigungsorgan (25) verbunden ist, das vom Verwender betätigt wird.

11. Vorrichtung nach Anspruch 9, bei der die Luftausstoßeinrichtung (20) einen Blasebalg (29) umfasst, der mit dem Lufteinlaß (11) des Behälters (10) verbunden ist.

12. Vorrichtung nach einem der Ansprüche 9 bis 11, bei der der Pulver-Auslaß (15) des Behälters (10) vorzugsweise in dichter Weise mit einem Austrittskanal (10) verbunden ist, dessen Durchmesser größer als der Durchmesser der Kugel (16) ist, wobei der Kanal (40) Anschlagseinrichtungen (41) für die Kugel (16) umfasst, um das Ausstoßen der Kugel aus der Vorrichtung beim Ausstoßen des Pulvers zu verhindern.

13. Vorrichtung nach einem der Ansprüche 9 bis 12, bei der der Lufteinlaß (11) des Behälters (10) durch eine für das Pulver und die Luft dichte Verschlußmembran (13) verschlossen ist, wobei die Vorrichtung Öffnungseinrichtungen (23) für den Lufteinlaß (11) des Behälters (10) umfasst, die geeignet sind, die Membran (13) vor dem Ausstoßen der Pulver-Dosis zu öffnen.

14. Vorrichtung nach Anspruch 13, bei der die Öffnungseinrichtungen (23) für den Lufteinlaß (11 ) des Behälters (10) eine Durchbohrungs-Hohlnadel (23) umfassen, die geeignet ist, die Verschlußmembran (13) zu durchstoßen und den Behälter (10) mit der Luftausstoßeinrichtung (20) zu verbinden.

15. Vorrichtung nach einem der Ansprüche 9 bis 14, bei der die Luftausstoßeinrichtung (20) Rückstelleinrichtungen (26) derart umfasst, daß die Vorrichtung wieder geladen und/oder wiederverwendet werden kann.

16. Vorrichtung nach den Ansprüchen 10 und 15, bei der die Rückstelleinrichtungen eine Feder (26) umfassen, die den Kolben (21) und das Betätigungsorgan (25) in ihre jeweiligen Ruhelagen zurückbewegt.

17. Vorrichtung nach einem der Ansprüche 9 bis 16, bei der der Behälter (10) in entfernbarer Weise in der Vorrichtung derart montiert ist, daß er nach jeder Betätigung der Vorrichtung ausgetauscht werden kann.

18. Vorrichtung nach einem der Ansprüche 9 bis 17, bei der vor der Betätigung die gesamte Vorrichtung in einer luftdichten Verpackung verpackt ist.

19. Verfahren zum Füllen eines Pulverbehälters nach Anspruch 7 oder 8, **dadurch gekennzeichnet, daß** es folgende Schritte umfasst:
- Einführen (A) einer Pulver-Dosis in die Buchse (18) durch die Ausgangsöffnung für das Pulver (15),
- Aufpressen (B) der Kappe (19) um die Buchse (18), wobei die Kappe (19) die Verschlußkugel (16) enthält, die von den Einrichtungen (41) zum Befestigen der Kugel festgehalten wird, und
- Einführen (C) der Kugel (16) in die Ausgangsöffnung (15) für das Pulver der Buchse (18).
